# FASCICULE DE BREVET EUROPEEN

(11) **EP 0 502 766 B1**
(45) Date de publication et mention de la délivrance du brevet: **10.07.1996**
(21) Numéro de dépôt: 92400520.0
(22) Date de dépôt: 28.02.1992
(51) Int. Cl.: A23J 7/00, A61K 35/60

(54) **Compositions diététiques à base de lipides phosphorylés et leur utilisation dans l'amélioration des troubles de la vision**
Diätetische Zusammensetzungen aus phosphorylierten Lipiden und ihre Verwendung zur Besserung von Sehproblemen
Dietary compositions based on phosphorylated lipids and their use to improve vision troubles

(30) Priorité: 05.03.1991 FR 9102613
(43) Date de publication de la demande: 09.09.1992
(73) Titulaire: INSTITUT DE RECHERCHE BIOLOGIQUE S.A., F-78870 Bailly (FR)
(72) Inventeur: Ponroy, Yves, F-78890 Garancières (FR); Forgeot, Marcel, F-78720 Dampierre en Yvelines (FR); Fraisse, Pierrette, F-34000 Montpellier (FR)
(74) Mandataire: Burtin, Jean-François

(56) Documents cités:
- EP-A- 0 180 786
- EP-A- 0 312 814
- EP-A- 0 410 749
- WO-A-90/11079
- DE-A- 1 932 918
- FR-A- 2 625 875
- US-A- 3 546 337
- US-A- 4 981 681
- PROCEEDINGS OF THE NATIONAL ACADEMY OF SCIENCES OF USA vol. 83, Juin 1986, WASHINGTON US pages 4021 - 4025; MARTHA NEURINGER: 'Biochemical and functional effects of prenatal and postnatal w3 fatty acid deficiency on retina and brain in rhesus monkeys'
- WORLD PATENTS INDEX LATEST Week 8545, Derwent Publications Ltd., London, GB; AN 85-280928 45
- WORLD PATENTS INDEX LATEST Week 8610, Derwent Publications Ltd., London, GB; AN 86-064833
- WORLD PATENTS INDEX LATEST Week 9113, Derwent Publications Ltd., London, GB; AN 91-089022 13
- WORLD PATENTS INDEX LATEST Week 9110, Derwent Publications Ltd., London, GB; AN 91-070562 10
- CHEMICAL ABSTRACTS, vol. 108, no. 1, 4 Janvier 1988, Columbus, Ohio, US; abstract no. 5162N, DIANA JANE PHILBRICK ET AL.: 'Ingestion of fish oil or a derived n-3 fatty acid concentrate containing eicosapentaenoic acid (EPA) affects fatty acid compositions of individual phospholipids of rat brain, sciatic nerve and retina.' & J. Nutr. vol. 117 no. 10 1987 pages 1663-70' page 494 ;colonne 1 ;

## Description

La présente invention se rapporte à de nouvelles compositions diététiques riches en lipides phosphorés d'origine naturelle et leur utilisation pour l'amélioration des troubles visuels diffus.

Elle a plus particulièrement pour objet des compositions diétetiques renfermant des phospholipides cérébraux seuls ou en association avec des huiles d'origine animale riches en acides gras polyinsaturés de la série ω-3.

Elle a spécifiquement pour objet des compositions diététiques renfermant des phospholipides cérébraux dont les constituants principaux sont de formule :

### a) un triglycéride

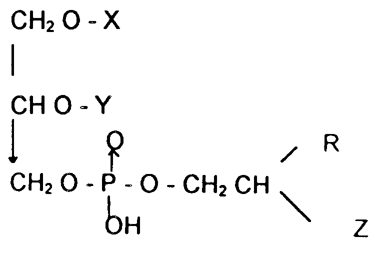

dans laquelle X et Y, identiques ou différents, représentent le reste acyle d'un acide gras polyinsaturé ayant de 18 à 24 atomes de carbone.

R est de l'hydrogène ou un carboxyle
et Z est un radical aminé, primaire, secondaire, tertiaire ou quaternaire
et

### b) de l'huile de poisson des mers froides, riche en DHA, en EPA et en vitamine A en association ou en mélange avec un diluant, un agent support et/ou un agent liant et un complément alimentaire.

On sait, en effet, que les phospholipides cérébraux jouent un rôle important dans la protection de la cellule nerveuse mais participent aussi aux activités membranaires et trans menbranaires. Cette fonction est particulièrement importante pour les membranes des cellules rétiniennes qui reçoivent la lumière, la transforment en influx nerveux par l'intermédiaire du pigment photorécepteur (Rhodopsines) et transmettent cet influx nerveux aux autres cellules nerveuses. Les compositions selon l'invention apporte des phospholipides d'origine cérébrale donc directement utilisables par l'organisme. Ils ont la propriété d'être riches en acides gras de poids moléculaire élevé, polyinsaturés et de parvenir au cerveau avec une bien meilleure efficacité que des phospholipides extraits de foie ou d'oeuf. Cet apport direct est particulièrement intéressant chez les personnes âgées dont les systèmes enzymatiques donnant naissance à des acides gras polyinsaturés peuvent être déficients et par voie de conséquence entraînent des troubles de la vision.

Les huiles de poisson des mers froides sont des huiles d'expression de morue, de fletan, de hareng ... Elles sont riches en DHA. Or, on a déterminé que les membranes des cellules en bâtonnet de la rétine contiennent jusqu'à 45% de DHA. En outre, les analyses histologiques de la rétine indiquent que les molécules de DHA se trouvent toujours à proximité des molécules du pourpre rétinien. Ceci peut mettre en évidence le fait que le DHA participe au processus de la vision. On a montré, en effet, (M. NEURINGER et al. Proceed. Natl. Acad. Sci. USA 83 (1986) 4021) que des carences en acides gras polyinsaturés étaient susceptibles de modifier la répartition des acides gras membranaires de la rétine et perturbaient l'activité bioélectrique de la rétine. Selon ces auteurs, le singe alimenté par des régimes alimentaires très pauvres en acides gras polyéthyléniques du type 18:3ω3 pendant la gestation ainsi que sa descendance, montrent des taux de phosphatidyl éthanolamine dans la rétine, moitié moindre que chez les sujets normaux et quatre fois moindre dans le cortex cérébral. Les animaux déficients ont une activité visuelle subnormale et surtout un temps de récupération prolongé de l'électrorétinogramme après séjour dans l'obscurité consécutivement à un flash saturant.

C'est donc l'indication qu'un apport en DHA est essentiel pour le développement et le maintien d'une bonne acuité visuelle et pour une activité bioélectrique normale de la rétine.

La référence Römpp (Chemie Lexikon p.2338-2339) décrit des lécithines ayant le groupe hydroxyle primaire du glycérol estérifié par un acide gras saturé et le groupe hydroxyle secondaire estérifié par un acide gras non saturé. Le cas de l'acide gras polyinsaturé n'est pas évoqué. C'est pourquoi l'indication fournie par la référence Rompp selon laquelle les lécithines ayant principalement des acides gras non saturés sont désignées comme phospholipides essentiels, ne préjugent en rien du fait qu'il existerait des phospholipides avec deux restes acyle dérivés d'acide gras non saturés et que ces acides gras non saturés sont polyinsaturés.

En effet, la transformation des acides gras non saturés dans l'organisme - surtout dans l'organisme des personnes âgées - est quasiment inexistant par suite de la disparition du matériel enzymatique approprié (Δ5 desaturase et Δ6 desaturase). C'est en ce sens là que l'apport de phospholipides cérébraux joue un rôle important car il fournit à l'organisme un apport en acides gras directement utilisables et qui sont essentiels pour maintenir ou améliorer la vision.

Il s'avérait donc nécessaire de rechercher et de trouver une lécithine qui réponde à ce critère d'utilisation directe.

La référence E. Pistolesi (brevet européen 0 180 786) décrit des compositions pharmaceutiques ou diététiques pour la prévention ou le traitement des pathologies du domaine vasculaire, de l'artériosclérose et des thrompathies qui renferment de la lécithine et des huiles riches en acide eicosapentaenoïque et/ou docosahexaenoïque.

Dans cette référence, la lécithine est d'origine naturelle (soja, arachides, oeufs, tissus animals) et/ou synthétique, de préférence celles ayant une teneur en phosphatidylcholine. Les acides polyéniques contenus dans ces compositions sont trouvés dans l'huile de foie de morue. Les deux constituants sont mélangés par dissolution ou bien administrés séparément et à des intervalles distincts.

Dans cette association, la lécithine sert essentiellement de vecteur pour apporter de l'huile de foie de morue et en dissimuler la saveur. Les modifications apportées à l'état de la paroi des vaisseaux ou les changements dans l'agrégation plaquettaire résultent essentiellement de l'incorporation des acides gras polyinsaturés dont on connaît par ailleurs le rôle protecteur ou prophylactique dans les risques cardiovasculaires.

Il était donc désirable d'associer ces deux constituants dans les compositions diététiques de l'invention pour usage bien différent qui consiste à prévenir ou à traiter les troubles de la vision.

Les compositions diététiques selon l'invention peuvent contenir, en outre, un ou des anthocyanosides comme ceux contenus dans les extraits de myrtille, qui contribuent à améliorer l'acuité visuelle et principalement l'acuité visuelle crépusculaire ou nocturne.

Les anthocyanosides, en effet, exercent un effet favorable sur l'acuité visuelle en favorisant la régénération du pourpre rétinien et en améliorant la résistance capillaire des petits vaisseaux de la rétine tout en diminuant leur perméabilité.

L'extrait de myrtille riche en anthocyanosides apporte, de ce fait, un élément favorable pour l'intégrité de la vision.

Les compositions diététiques de l'invention peuvent aussi renfermer une préparation riche en caroténoides comme par exemple l'oléo-résine de carotte. Celle-ci est un produit naturel qui contient du β-caroteine ou provitamine A. C'est un précurseur de la vitamine A. Cette vitamine joue un rôle considérable dans le maintien de la vision crépusculaire et constitue le principe actif de base pour la réformation constante du pourpre rétinien décomposé par les faisceaux lumineux.

Les compositions diététiques selon l'invention peuvent aussi contenir des tocophérols, notamment sous forme de concentrés de germes de céréales comme l'huile de germe de blé. Les tocophérols empêchent ou retardent l'oxydation et l'hydropéroxydation des acides gras polyinsaturés.

Comme excipients ou diluants inertes, on utilise de préférence des produits minéraux comme la silice, la terre d'infusoires, des carbonates alcalino-terreux. On peut également incorporer des agents liants en alimentaires comme des lécithines et notamment de la lécithine de soja.

La teneur en principes actifs dans les compositions diététiques peut varier dans des proportions importantes et dans des rapports importants. C'est ainsi que la teneur en huile de poisson des mers froides peut varier de 0,150 g à 0,300 g selon la concentration en acides gras polyinsaturés. Elle sera de préférence comprise entre 0,2 g et 0,3 g dans une préparation unitaire.

La teneur en phospholipides cérébraux est également fonction de la teneur en glycérides phosphorylés. Elle pourra s'échelonner de 10 à 100 mg et de préférence de 15 à 50 mg.

Pour les mêmes raisons, la teneur en extrait de myrtilles peut varier de 40 à 200 mg et de préférence de 60 à 100 mg.

La teneur en caroténoides sous forme d'oléo résine de carotte varie de 50 à 300 mg et de préférence de 80 à 120 mg.

La teneur en tocophérols pourra varier selon la richesse de l'huile végétale. Elle s'échelonnera de 3 à 10 mg en concentré d'huile végétale.

D'une manière particulièrement appropriée, les compositions diététiques selon l'invention sont formulées sous forme de poudres ou de granulés répartis en sachets ou en gélules.

D'une manière préférée, les compositions diététiques selon l'invention sont présentées sous forme de gélules d'un poids total fini à 800 mg.

L'administration se fait à raison de 2 à 4 capsules, de préférence 3 capsules, par jour à absorber avec un peu d'eau. La répartition des prises est laissée à la convenance du sujet soit le matin, soit le soir.

La durée de l'administration est de 2 à 3 mois.

Les compositions diététiques visent à corriger les déficiences de l'alimentation ou du métabolisme conduisant à des troubles visuels diffus (fatigue visuelle) chez des sujets dont l'acuité visuelle est amoindrie en raison de leurs activités mais ne présentent pas de pathologie ophtalmologique ou optique.

Le principal critère retenu lors de la détermination des propriétés des compositions a été la résistance à l'éblouissement après éblouissement central de la rétine. Les valeurs obtenues montrent que les sujets présentent une récupération plus rapide de l'acuité visuelle. Le gain total de l'acuité visuelle est proche de 1/10. En outre, l'acuité visuelle après éblouissement est récupérée 40 secondes plus tôt que chez des sujets ne recevant pas les compositions diététiques selon l'invention.

Enfin, les compositions diététiques selon l'invention quoique destinées à l'administration digestive, peuvent avoir un effet favorable sur l'évolution des maculopathies dégénératives, contrairement à ce qui est indiqué dans l'article de M.VARGA et coll (Klin. Mol. Augenheilk. 188 (1986) 622) lors de l'expérimentation par voie parentérale d'une préparation stérile de phosphatides rétiniens de porc. Les exemples suivants illustrent l'invention:

### EXEMPLE I

### Gélule à base d'huile de poissons des mers froides

| | |
|---|---|
| Huile de poissons des mers froides | 0,222 kg |
| Phospholipides cérébraux | 0,016667 kg |
| Extrait de myrtille | 0,07 kg |
| Oleorésine de carottes | 0,100 kg |
| Concentré d'huile végétale riche en Tocophérols | 0,005 kg |

pour 100 capsules finies au poids moyen de 0,800 g.

### EXEMPLE II

### Etude des compositions diététiques dans la fatigabilité visuelle

Le nombre total de sujets qui s'est prêté à cette expérience était de 17 dont 7 hommes et 10 femmes. L'âge moyen des sujets était de 52 ± 13,5 ans. Tous les sujets présentaient des troubles à type de fatigue visuelle dont l'ancienneté s'échelonnait de 3 à 36 mois avec une moyenne de 12,8 ± 10,4 mois. Elle se manifestait pendant le travail quotidien ou pendant le temps de lecture dont la durée moyenne était de 6,8 ± 2,2 heures.

Les compositions diététiques ont été utilisées à raison de 3 capsules par jour prises soit le matin, soit le soir. La durée de l'administration a varié de 2 à 3 mois avec une moyenne de 2,2 ± 0,3 mois. Aucun autre produit dont les effets auraient pu interférer avec les résultats n'a été associé pendant la durée de cet essai.

### RESULTATS

### a) Appréciation subjective

L'appréciation globale par le sujet lui-même a été formulée en fin d'administration. Les résultats ont été les suivants :

| | |
|---|---|
| - amélioré | 5 cas |
| - légèrement amélioré | 6 cas |
| - sans changement | 8 cas |

### b) Critères objectifs

Etude de la résistance à l'éblouissement par mesure de la récupération visuelle après éblouissement central de la rétine en fonction du temps.

Les mesures ont été effectuées sur l'oeil droit et sur l'oeil gauche de chaque sujet. Les résultats ont été ensuite rassemblés (calcul de la moyenne de récupération de l'acuité visuelle des deux yeux pour chaque patient). Les résultats ont été les suivants :

| | **AVANT TRAITEMENT** | **APRES TRAITEMENT** |
|---|---|---|
| T1 10 sec. | 0 ± 0 | 0 ± 0 |
| T2 20 sec. | 0 ± 0 | 0,007 ± 0,01 |
| T3 30 sec. | 0,012 ± 0,02 | 0,037 ± 0,04 |
| T4 40 sec. | 0,300 ± 0,04 | 0,077 ± 0,06 |
| T5 50 sec. | 0,050 ± 0,05 | 0,108 ± 0,07 |
| T6 60 sec. | 0,085 ± 0,08 | 0,137 ± 0,07 |
| T7 70 sec. | 0,104 ± 0,08 | 0,166 ± 0,09 |
| T8 80 sec. | 0,125 ± 0,09 | 0,192 ± 0,08 |
| T9 90 sec. | 0,146 ± 0,10 | 0,214 ± 0,09 |
| T10 100 sec. | 0,167 ± 0,10 | 0,238 ± 0,10 |
| T11 110 sec. | 0,179 ± 0,11 | 0,158 ± 0,10 |
| T12 120 sec. | 0,195 ± 0,12 | 0,270 ± 0,10 |

On constate donc une évolution favorable en fonction du temps. La récupération de l'acuité visuelle est plus précoce : des performances plus importantes sont obtenues et leur apparition est plus rapide.

Les résultats obtenus à chaque mesure, comparés à l'aide du test t de Student sur séries appariées sont statistiquement significatifs de T2 à T12.

Au total :
- amélioration des performances visuelles après éblouissement central de la rétine : en fin de traitement, le gain total de l'acuité visuelle est proche de 1/10ème (0,075).
- rapidité d'obtention des performances visuelles après éblouissement central de la rétine : l'acuité visuelle maxima atteinte avant traitement est récupérée 40 secondes plus tôt après le traitement.

* en ce qui concerne la mesure de la récupération visuelle après éblouissement latéral, le gain total en fin de traitement est de :
   - 0,7 pour l'oeil droit (différence non significative)
   - 1 pour l'oeil gauche (différence statistiquement significative)
Les autres paramètres mesurés :
- vision nocturne
- sensibilité aux contrastes
- électrorétinogramme
- potentiels évoqués visuels
ont subit des modifications variables d'un sujet à l'autre et globalement non significatives.

### c) Tolérance clinique

Elle a été excellente chez tous les sujets.

### DISCUSSION et CONCLUSION

La sidération rétinienne produite par une luminance intense est le résultat de désordres photochimiques se produisant au niveau des cellules sensorielles réceptrices.

Les fortes luminances détruisent le pourpre rétinien et les pigments visuels. La reprise de l'activité rétinienne normale après cet éblouissement serait liée à la reconstitution de ces pigments.

## Revendications

1. Nouvelles compositions diététiques à base de phospholipides cérébraux caractérisés en ce qu'elles renferment à titre de constituants principaux un triglycéride de formule : 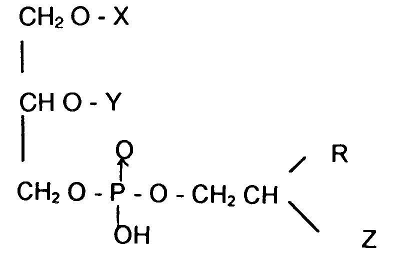 dans laquelle X et Y, identiques ou différents, représentent le reste acyle d'un acide gras polyinsaturé ayant de 18 à 24 atomes de carbone
R est un hydrogène ou un carboxyle
et Z est un radical aminé primaire, secondaire, tertiaire ou quaternaire et de l'huile de poisson des mers froides
en association ou en mélange avec un diluant, un agent support et/ou un agent liant et un complément alimentaire, approprié pour l'administration par voie digestive.

2. Une composition diététique selon la revendication 1, qui contient en outre un ou des anthocyanosides, une préparation riche en caroténoïdes et des tocophérols.

3. Une composition diététique selon l'une des revendications 1 ou 2, dans laquelle la teneur en huile de poisson des mers froides varie de 0,150 à 0,300 g, la teneur en phospholipides cérébraux de 0,010 à 0,100 g et la teneur en extrait de myrtille de 0,040 à 0,200 g par prise unitaire.

4. Utilisation des compositions diététiques selon l'une des revendications 1 à 3, en vue de la réalisation d'un produit visant à corriger les déficiences de l'alimentation ou du métabolisme conduisant à des troubles visuels diffus comme la fatigue visuelle.

5. Utilisation des compositions diététiques selon l'une des revendications 1 à 3, en vue de la réalisation d'un produit amenant la récupération de l'acuité visuelle après éblouissement.

6. Utilisation des compositions diététiques selon l'une des revendications 1 à 3, en vue de la réalisation d'un produit ayant un effet favorable sur l'évolution des maculopathies rétiniennes.

## Claims

1. Novel dietetic compositions based on brain phospholipids wherein they contain as main constituent a triglyceride having the formula 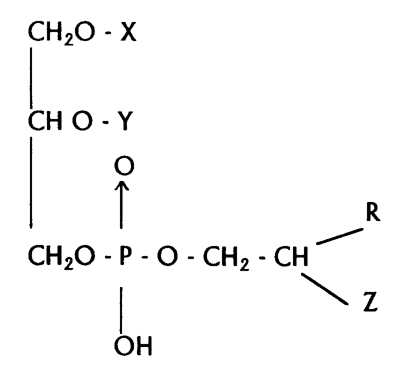 in which X and Y, the same or different, represent the acyl moiety of a polyunsaturated fatty acid having from 18 to 24 carbon atoms
R is a hydrogen or a carboxyl
and Z is a primary, secondary, tertiary or quaternary amino radical
and fish oil from cold sea
in association or in admixture with a diluent, a carrier and/or a binding agent and a nutritional complement, suitable for the digestive way.

2. A dietetic composition according to claim 1, which contains, moreover, one or some anthocyanosids, a preparation rich in carotenoids and tocopherols.

3. A dietetic composition according to any of claims 1 or 2, wherein the content in fish oil from cold sea ranges from 0,150 to 0,300 g, the content in brain phospholipids ranges from 0,010 to 0,100 g and the content in bilberry extract ranges from 0,040 to 0,200 g per unit dosage.

4. Use of dietetic compositions according to any of claims 1 to 3, for the realization of a product suitable to correct the alimentary or metabolism deficiencies leading to diffuse eye trouble such as visual tiredness.

5. Use of dietetic compositions according to any of claims 1 to 3, for the realization of a product supplying the recuperation of the visual acuteness after dazzle.

6. Use of dietetic compositions according to any of claims 1 to 3, for the realization of a product having a favourable effect on the evolution of retinal maculopathias.

## Patentansprüche

1. Neue diätetische Zusammensetzungen aus Gehirnphospholipiden dadurch gekennzeichnet dass sie, als Hauptwirkstoffe ein Triglyzerid, der Formel 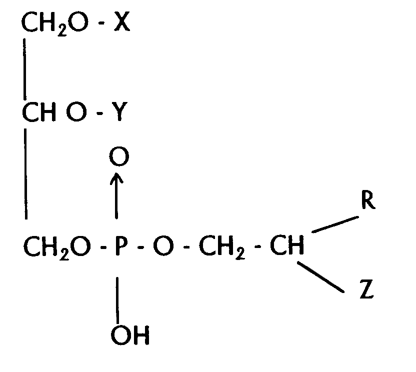 in der X und Y, identische oder unterschiedliche sind, für eine Acylrest von einer polyungesättigte Fettsaüre steht, mit 18 bis 24 Kohlenstoff Atomen
R einen Wasserstoffatom oder ein Carbonsaüre ist
und Z einen primären, sekundären, tertiären oder quaternären Aminoradikal ist zusammen mit einem Fischöl die von kalt See auskommt, enthälten in Verbindung oder Vermischung mit einem Verdünnungsmittel, einem Träger und/oder ein Bindemittel und Ernährungszusatz, der für die Verabreichung durch verdauungs Weg geeignet sind.

2. Ein diätetische Zusammensetzung nach Anspruch 1, der außerdem ein oder mehrere Anthocyanoside, eine an Carotenoide reich Zusammensetzung und Tocopherole enthält.

3. Ein diätetische Zusammensetzung nach einer der Ansprüche 1 oder 2, worin der Gehalt an Fischöl der kalt See, von 0,150 bis 0,300 g, der Gehalt an Gehirnphospholipiden von 0,010 bis 0,100 g and der Gehalt an Heidelbeere Extrakt von 0,040 bis 0,200 g per Verabreichungseinheit liegt.

4. Verwendung von diätetische Zusammensetzungen nach einer der Ansprüche 1 bis 3, für die Herstellung eines Produktes für die Verbesserung der Ernährung oder des Stoffwechsels, der zu zerstreute Sehstörungen als Sehmüdigkeit führt.

5. Verwendung von diätetische Zusammensetzungen nach einer der Ansprüche 1 bis 3, für die Herstellung eines Produkt der die Ruckgewinnung von der Sehschärfe nach Flimmern bringt.

6. Verwendung von diätetische Zusammensetzungen nach einer der Ansprüche 1 bis 3, für die Herstellung eines Produktes der die eine günstige Wirkung auf den Verlauf des Makulopathia des Netzhautes hat.
